# EUROPEAN PATENT SPECIFICATION

(11) **EP 3 365 064 B1**
(45) Date of publication and mention of the grant of the patent: **20.11.2019**
(21) Application number: 16787685.3
(22) Date of filing: 18.10.2016
(51) Int. Cl.: A61Q 5/02, A61K 8/42, A61K 8/55

(54) **SHAMPOO WITH A COOLING SENSATION COMPOUND**
SHAMPOO MIT EINER VERBINDUNG DIE EIN KÄLTEGEFÜHL VERMITTELT
SHAMPOOING AVEC COMPOSÉ DE SENSATION DE REFROIDISSEMENT

(30) Priority: 22.10.2015 US 201514920105
(43) Date of publication of application: 29.08.2018
(73) Proprietor: The Procter & Gamble Company, Cincinnati, OH 45202 (US)
(72) Inventor: RENOCK, Sean, Michael, Cincinnati, Ohio 45202 (US); HAUGHT, John, Christian, Cincinnati, Ohio 45202 (US)
(74) Representative: P&G Patent Germany
(86) International application number: PCT/US2016/057477
(87) International publication number: WO 2017/070083

(56) References cited:
- WO-A1-2008/075942
- WO-A2-2013/014235
- US-A- 4 136 163

## Description

### FIELD OF THE INVENTION

The present invention relates to hair care shampoo composition containing a perfume system comprising one or more coolants, wherein the cool sensation provided by the coolant is enhanced in terms of quicker onset, greater intensity, and/or longer duration, thereby improving appeal and acceptability of the compositions to consumers.

### BACKGROUND OF THE INVENTION

Hair care shampoo rinse off products are commercially available. Further, anti-dandruff shampoos typically incorporate an anti-dandruff active. One type of anti-dandruff agents are particulate, crystalline anti-dandruff agents, such as sulfur, selenium disulfide and heavy metal salts of pyridinethione. Soluble anti-dandruff agents, such as ketoconazole, are also available.

Sensates, such as menthol, have been delivered as soluble actives from hair care formulations to provide cooling scalp relief while delivering the sensation of a fresh and clean scalp. The scalp cooling sensation that menthol can deliver via a shampoo remains for a short amount of time after shampooing (1-60min). Consumers have expressed that they would enjoy longer lasting cooling throughout the day especially in hot and dusty climates. This is currently a need which cannot be provided from current shampoo formulations in market. In addition, although some consumers like the peppermint smell that menthol delivers, some consumers describe the smell, in a negative context, as metallic or medicinal. The present invention can be utilized to provide longer lasting cooling throughout the day while providing no smell to the consumer. This compounds ability to deliver longer lasting cooling can convey a signal of efficacy to the consumer that this material is really working vs. their current menthol shampoo. This material, which provides no smell character, can be formulated with fragrances that the consumer may find more pleasing for a long term cooling / clean feel vs. the peppermint medicinal smell of menthol.

A large number of coolant compounds of natural or synthetic origin have been described. The most well-known compound is menthol, particularly 1-menthol, which is found naturally in peppermint oil, notably of Mentha arvensis L and Mentha viridis L. Of the menthol isomers, the 1-isomer occurs most widely in nature and is typically what is referred by the name menthol having coolant properties. L-menthol has the characteristic peppermint odor, has a clean fresh taste and exerts a cooling sensation when applied to the skin and mucosal surfaces. Other isomers of menthol (neomenthol, isomenthol and neoisomenthol) have somewhat similar, but not identical odor and taste, i.e., some having disagreeable notes described as earthy, camphor, musty. The principal difference among the isomers is in their cooling potency. L-menthol provides the most potent cooling, i.e., having the lowest cooling threshold of about 800 ppb, i.e., the concentration where the cooling effect could be clearly recognized. At this level, there is no cooling effect for the other isomers. For example, d-neomenthol is reported to have a cooling threshold of about 25,000 ppb and 1-neomenthol about 3,000 ppb. (R. Emberger and R. Hopp, "Synthesis and Sensory Characterization of Menthol Enantiomers and Their Derivatives for the Use in Nature Identical Peppermint Oils," Specialty Chemicals (1987), 7(3), 193-201). This study demonstrated the outstanding sensory properties of 1-menthol in terms of cooling and freshness and the influence of stereochemistry on the activity of these molecules.

Among synthetic coolants, many are derivatives of or are structurally related to menthol, i.e., containing the cyclohexane moiety, and derivatized with functional groups including carboxamide, ketal, ester, ether and alcohol. Examples include the p-menthanecarboxamide compounds, such as N-ethyl-p-menthan-3-carboxamide, known commercially as "WS-3", and others in the series, such as WS-5 (N-ethoxycarbonylmethyl-p-menthan-3-carboxamide), WS-12 [N-(4-methoxyphenyl)-p-menthan-3-carboxamide] and WS-14 (N-tert-butyl-p-menthan-3-carboxamide). Examples of menthane carboxy esters include WS-4 and WS-30. An example of a synthetic carboxamide coolant that is structurally unrelated to menthol is N,2,3-trimethyl-2-isopropylbutanamide, known as "WS-23". Additional examples of synthetic coolants include alcohol derivatives such as 3-(1-menthoxy)-propane-1,2-diol known as TK-10, isopulegol (under the tradename Coolact P) and p-menthane-3,8-diol (under the tradename Coolact 38D); menthone glycerol acetal known as MGA; menthyl esters such as menthyl acetate, menthyl acetoacetate, menthyl lactate known as Frescolat® supplied by Haarmann and Reimer, and monomenthyl succinate under the tradename Physcool from V. Mane. TK-10 is described in U.S. Pat. No. 4,459,425 to Amano et al. Other alcohol and ether derivatives of menthol are described e.g., in GB 1,315,626 and in U.S. Pat. Nos. 4,029,759; 5,608,119; and 6,956,139. WS-3 and other carboxamide cooling agents are described for example in U.S. Pat. Nos. 4,136,163; 4,150,052; 4,153,679; 4,157,384; 4,178,459 and 4,230,688. Additional N-substituted p-menthane carboxamides are described in WO 2005/049553A1 including N-(4-cyanomethylphenyl)-p-menthanecarboxamide, N-(4-sulfamoylphenyl)-p-menthanecarboxamide, N-(4-cyanophenyl)-p-menthanecarboxamide, N-(4-acetylphenyl)-p-menthanecarboxamide, N-(4-hydroxymethylphenyl)-p-menthanecarboxamide and N-(3-hydroxy-4-methoxyphenyl)-p-menthanecarboxamide. Other N-substituted p-menthane carboxamides include amino acid derivatives such as those disclosed in WO 2006/103401 and in US Pat. Nos. 4,136,163; 4,178,459 and 7,189,760 such as N-((5-methyl-2-(1-methylethyl)cyclohexyl)carbonyl)glycine ethyl ester and N-((5-methyl-2-(1-methylethyl)cyclohexyl)carbonyl)alanine ethyl ester. Menthyl esters including those of amino acids such as glycine and alanine are disclosed e.g., in EP 310 299 and in U.S. Pat. Nos. 3,111,127; 3,917,613; 3,991,178; 5,703,123; 5,725,865; 5,843,466; 6,365,215; 6,451,844; and 6,884,903. Ketal derivatives are described, e.g., in U.S. Pat. Nos. 5,266,592; 5,977,166 and 5,451,404. Additional agents that are structurally unrelated to menthol but have been reported to have a similar physiological cooling effect include alpha-keto enamine derivatives described in U.S. Pat. No. 6,592,884 including 3-methyl-2-(1-pyrrolidinyl)-2-cyclopenten-1-one (3-MPC), 5-methyl-2-(1-pyrrolidinyl)-2-cyclopenten-1-one (5-MPC), and 2,5-dimethyl-4-(1-pyrrolidinyl)-3(2H)-furanone (DMPF); icilin (also known as AG-3-5, chemical name 1-[2-hydroxyphenyl]-4-[2-nitrophenyl]-1,2,3,6-tetrahydropyrimidine-2-one) described in Wei et al., J. Pharm. Pharmacol. (1983), 35:110-112. Reviews on the coolant activity of menthol and synthetic coolants include H. R. Watson, et al. J. Soc. Cosmet. Chem. (1978), 29, 185-200 and R. Eccles, J. Pharm. Pharmacol., (1994), 46, 618-630.

The present invention provides shampoo compositions comprising of one or more coolants that can be used in shampoo, wherein the cooling and refreshing sensation provided by the coolants (s) is potentiated in terms of onset, intensity, and / or duration. In an embodiment, the present invention provides anti-dandruff compositions comprising of one or more coolants that can be used in shampoo, wherein the cooling and refreshing sensation provided by the coolants (s) is potentiated in terms of onset, intensity, and / or duration.

### SUMMARY OF THE INVENTION

The present invention is directed to a hair care shampoo composition comprising a compound comprising the following structure: 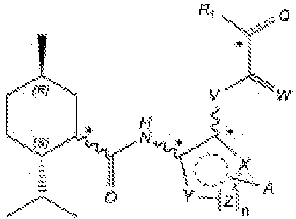
R₁ is selected from H, alkyl, amino alkyl, alkoxy;
Q = H₂, O, -OR₁, -N(R₁)₂, -OPO(OR₁)ₓ, -PO(OR₁)ₓ, -P(OR₁)ₓ where x = 1-2;
V = NR₁, O, -OPO(OR₁)ₓ, -PO(OR₁)ₓ, -P(OR₁)ₓ where x = 1-2;
W = H₂, O;
X, Y = independently selected from H, aryl, naphthyl for n=0;
X, Y = aliphatic CH₂ or aromatic CH for n ≥ 1 and Z is selected from aliphatic CH₂, aromatic CH, or heteroatom;
A = aryl, subsitituted aryl or fused aryl; and stereochemistry is variable at the positions marked*.

These and other features, aspects, and advantages of the present invention will become evident to those skilled in the art from the detailed description that follows.

### DETAILED DESCRIPTION OF THE INVENTION

The present invention is directed to the discovery that certain cyclohexanecarboxamide structures deliver the means to drive a cooling response at low concentrations. It has been discovered that cyclohexanecarboxamide, 5-methyl-2-(1-methylethyl)-*N*-(2-phenylethyl)-, (1*R,*2*S,*5*R*) (CAS# 824947-52-6) and cyclohexanecarboxamide, 5-methyl-2-(1-methylethyl)-N-(2-phenylethyl)-, (1R,2S,5R) (CAS# 847564-71-0) structures with 2-amino-propanamide (CAS# 4726-84-5) have enhanced long lasting cooling properties and cyclohexanecarboxamide, 5-methyl-2-(1-methylethyl)-N-phenyl-, (1R,2S,5R) and cyclohexanecarboxamide, 5-methyl-2-(1-methylethyl)-N-1-naphthalenyl-(1R,2S,5R) (CAS# 863091-95-6) structures with an aminoethane (CAS# 75-04-7) moiety deliver a warming sensation. Both types of cyclohexanecarboxamide (cooling and warming) are efficacious at low use levels (1-10 ppm). The stereochemistry assigned to the compounds above is based on the dominant isomer (1R, 2S, 5R) derived from the menthol starting material. One or more additional isomers and/or enantiomers may occur due to the additional chiral sites built out from the amide linkage.

Structures built off of the cyclohexanecarboxamide backbone have been applied as anti-cancer agents as disclosed in WO 2009/067410. As shown in US Pat. No. 4,150,052, only a select few of the cyclohexanecarboxamide derivatives had noticeable cooling. The molecules disclosed in WO 2009/067410 were evaluated for their TRPM8 activity in relation to the destruction of prostate cancer cells. Thus cooling would have been an undesirable effect and something they would have avoided.

The present invention is thus based on the discovery that select molecules can be used to drive a cooling response when formulated into shampoo products. A second object of this invention shows the discovery that select cyclohexanecarboxamide derivatives can provide long lasting cooling at very low levels, allowing for formulation efficiencies, in particular coolant compounds (coolants), such as described below.

### DETAILED DESCRIPTION OF EMBODIMENTS OF THE INVENTION

While the specification concludes with claims which particularly point out and distinctly claim the invention, it is believed that the provided invention will be better understood from the following description.

In all embodiments of the present invention, all percentages and ratios used herein are by weight of the total composition, unless otherwise designated. All measurements are understood to be made at ambient conditions, where "ambient conditions" means conditions at about 25 °C, under about one atmosphere of pressure, and at about 50% relative humidity (RH), unless otherwise designated. All numeric ranges are inclusive of narrower ranges; delineated upper and lower range limits are combinable to create further ranges not explicitly delineated. All numerical amounts are understood to be modified by the word "about" unless otherwise specifically indicated. All weights and % weights as they pertain to listed ingredients are based on the active level and do not include carriers or by-products that may be included in commercially available materials, unless otherwise specified. The number of significant digits conveys neither a limitation on the indicated amounts nor on the accuracy of the measurements. The term "molecular weight" or "M.Wt." as used herein refers to the weight average molecular weight unless otherwise stated. The weight average molecular weight may be measured by gel permeation chromatography. "QS" means sufficient quantity for 100%.

The term "comprising", as used herein, means that other steps and other ingredients which do not affect the end result can be added. The term encompases the terms "consisting of" and consisting essentially of". The compositions, methods and processes of the present invention can comprise, consist essentially of, or consist of, the elements and limitations of the invention described herein, as well as any of the additional or optional ingredients, components, steps, or limitations described herein.

"Dermatologically acceptable" means that the compositions or components described are suitable for use in contact with human skin tissue without undue toxicity, incompatibility, instability, allergic response, and the like.

"Safe and effective amount" means an amount of a compound or composition sufficient to significantly induce a positive benefit.

"Soluble" means at least about 0.1 g of solute dissolves in 100 ml of solvent, at 25 °C and 1 atm of pressure.

The term "substantially free from" or "substantially free of' as used herein means less than about 1%, or less than about 0.8%, or less than about 0.5%, or less than about 0.3%, or about 0%, by total weight of the composition.

"Hair," as used herein, means mammalian hair including scalp hair, facial hair and body hair, particularly on hair on the human head and scalp.

"Cosmetically acceptable," as used herein, means that the compositions, formulations or components described are suitable for use in contact with human keratinous tissue without undue toxicity, incompatibility, instability, allergic response, and the like. All compositions described herein which have the purpose of being directly applied to keratinous tissue are limited to those being cosmetically acceptable.

"Derivatives," as used herein, includes but is not limited to, amide, ether, ester, amino, carboxyl, acetyl, acid, salt and/or alcohol derivatives of a given compound.

"Polymer," as used herein, means a chemical formed from the polymerisation of two or more monomers. The term "polymer" as used herein shall include all materials made by the polymerisation of monomers as well as natural polymers. Polymers made from only one type of monomer are called homopolymers. Polymers made from two or more different types of monomers are called copolymers. The distribution of the different monomers can be calculated statistically or block-wise - both possibilities are suitable for the present invention. Except if stated otherwise, the term "polymer" used herein includes any type of polymer including homopolymers and copolymers.

All percentages and ratios used hereinafter are by weight of total composition, unless otherwise indicated. All percentages, ratios, and levels of ingredients referred to herein are based on the actual amount of the ingredient, and do not include solvents, fillers, or other materials with which the ingredient may be combined as a commercially available product, unless otherwise indicated.

All measurements referred to herein are made at 25°C unless otherwise specified.

Ideally, a coolant can produce a cooling or freshness sensation similar to that produced by menthol, but without certain of the disadvantages associated with menthol, such as flavor modification, bitter aftertaste, off-flavor, strong odor and burning or irritating sensation, particularly at high concentrations. It is desirable that the coolant compounds barely possess a distinctive odor or flavor while providing a pleasant fresh cool sensation of prolonged duration, in order that the effect can still be perceived for a considerable time after use, for example, longer than 15 minutes. Menthol generally provides an initial high cooling impact, but its effect is somewhat transient in that the cool sensation drops sharply within a few minutes after use. By contrast, a number of longer lasting coolant compounds may fail to provide an immediate cooling perception, i.e., within a few seconds of application, particularly when used at low levels. Thus there is a continuing need for means to potentiate the activity of coolant chemicals, in terms of quickening the onset of the cooling sensation, intensifying the cooling sensation, especially at lower concentrations, and producing a longer lasting sensation of cooling and freshness than what menthol provides.

As stated previously, the present invention is directed to the discovery that specific 5-methyl-2-(1-methylethyl)-*N*-(2-phenylethyl)-, (1*R*, 2*S*, 5*R*) cyclohexanecarboxamide structures, as shown below, deliver the means to drive a cooling response at low concentrations.

Structure I, which includes compounds of the present invention, as shown below, and which includes compound 28. Structure I represents a heteroalkyl substituted aryl or heteroalkyl-aryl substituted alkyl carboxamide of methanol having the shown below structure and including any acceptable salts or solvates thereof; wherein: 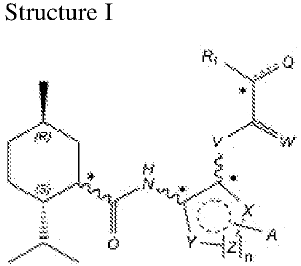
R₁ is selected from H, alkyl, amino alkyl, alkoxy;
Q = H₂, O, -OR₁, -N(R₁)₂, -OPO(OR₁)ₓ, -PO(OR₁)ₓ, -P(OR₁)ₓ where x = 1-2;
V = NR₁, O, -OPO(OR₁)ₓ, -PO(OR₁)ₓ, -P(OR₁)ₓ where x = 1-2;
W = H₂, O;
X, Y = independently selected from H, aryl, naphthyl for n=0;
X, Y = aliphatic CH₂ or aromatic CH for n ≥ 1 and Z is selected from aliphatic CH₂, aromatic CH, or heteroatom;
A = aryl, subsitituted aryl or fused aryl; and stereochemistry is variable at the positions marked*.

A number of stereoisomers are contemplated in the above Structure I, where substitution is allowed and the relative configuration of each stereo center will dictate the activity towards the receptor. While it is known that the stereochemistry of side chain groups may be important to the activity of the molecule, the activity of these compounds in vivo is highly unpredictable. In some cases, isomers of the same molecule may have comparable activity. In other cases, stereoisomers of the same molecule could have enhanced or diminished activity towards the receptor. In some cases, individual stereoisomers may have no activity.

Specific compounds of interest may derive from the 1R, 2S, 5R configuration found in natural (-)-menthol. In these cases, the stereoisomeric derivatives of 1R, 2S, 5R-menthyl carboxamide will be found in the substituted alkyl side chain fragment of the molecule. While the 1R, 2S, 5R configuration is known to be important to activity, the 1S,2S,5R neo-isomer of N-substituted menthyl carboxamide derivatives has also shown promise. 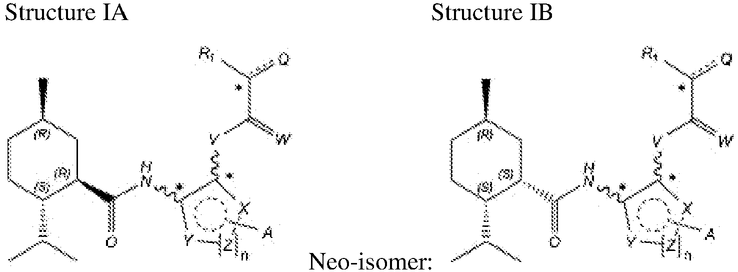
R₁ is selected from H, alkyl, amino alkyl, alkoxy;
Q = H₂, O, -OR₁, -N(R₁)₂, -OPO(OR₁)ₓ, -PO(OR₁)ₓ, -P(OR₁)ₓ where x = 1-2;
V = NR₁, O, -OPO(OR₁)ₓ, -PO(OR₁)ₓ, -P(OR₁)ₓ where x = 1-2;
W = H₂, O;
X, Y = independently selected from H, aryl, naphthyl for n=0;
X, Y = aliphatic CH₂ or aromatic CH for n ≥ 1 and Z is selected from aliphatic CH₂, aromatic CH, or heteroatom;
A = subsitituted aryl or fused aryl; and stereochemistry is variable at the positions marked*.

In the case of compounds 28 (and is discussed in more detail below), excellent activity is seen. In these cases, while not being limited to theory, specific activity among isomers is determined by the unique structural elements within the molecule in addition to the exact stereochemistry. While it is known that molecules having the right balance of hydrogen bonding groups (i.e. -NHR, -OH, -CONHR, etc.), Log P value, and molecular weight range are preferred, unique structural elements can contribute to activity within these preferred ranges. The current compounds of interest contain polar groups in the side-chain which are capable of both hydrogen bonding and balancing the lipophilicity of the overall structure. The stereochemical features within these molecules also impart a 3D dimensionality to the structure which can enhance interaction with specific receptors. It is believed that these unique structural features lead to enhanced affinity for the receptor which translates into the prolonged cooling effects which have been observed.

It has been discovered that cyclohexanecarboxamide, 5-methyl-2-(1-methylethyl)-*N*-(2-phenylethyl)-, (1*R*,2*S*,5*R*) (CAS# 824947-52-6) and cyclohexanecarboxamide, 5-methyl-2-(1-methylethyl)-N-(2-phenylethyl)-, (1R,2S,5R) (CAS# 847564-71-0) structures (shown above) with 2-amino-propanamide (CAS# 4726-84-5) have enhanced long lasting cooling properties and cyclohexanecarboxamide, 5-methyl-2-(1-methylethyl)-*N*-phenyl-, (1*R*,2*S*,5*R*) and cyclohexanecarboxamide, 5-methyl-2-(1-methylethyl)-N-1-naphthalenyl-(1*R*,2*S*,5*R*) (CAS# 863091-95-6) structures with an aminoethane (CAS# 75-04-7) moiety deliver a warming sensation. Both types of cyclohexanecarboxamide (cooling and warming) are efficacious at low use levels (1-10 ppm). The advantage of using such low levels of these materials allows for their formulation into higher water compositions, such as mouthrinses, without the need for additional processing aids, such as co-surfactants, oils, or other suspension agents. These materials may also provide mitigation of off tasting sensations, such as that derived from metal salts, peroxide, and CPC.

In a hair care shampoo composition, a level of approximately 4500ppm in product, has been found to be efficacious. In an embodiment, a range of about 4500 to about 100ppm, of the present compound may be used. It is assumed that materials when solubilized have approximate scalp deposition efficiency of 1%. It is believed that based on 1% depo efficiency that approximately about 45ppm to 1ppm would be deposited on scalp.

In an embodiment of the present invention, it may be hypothesized that, due to the long lasting cooling effects experienced by the consumer, once the cooling dissipates this could be a signal to the consumer that they may have to treat their scalp again with an anti-dandruff shampoo containing the sensate. This may improve compliance for use of the product and help to improve the consumers overall therapeutic response to the product.

### SHAMPOO COMPOSITION

The shampoo compositions of the present invention deliver, in addition to unique sensation, consumer desired cleansing and potentially conditioning.

### A. DETERSIVE SURFACTANT

The shampoo composition comprises one or more detersive surfactants, which provides cleaning performance to the composition. The one or more detersive surfactants in turn may comprise an anionic surfactant, amphoteric or zwitterionic surfactants, or mixtures thereof. Various examples and descriptions of detersive surfactants are set forth in U.S. Patent No. 6,649,155; U.S. Patent Application Publication No. 2008/0317698; and U.S. Patent Application Publication No. 2008/02063 55.

The concentration of the detersive surfactant component in the shampoo composition should be sufficient to provide the desired cleaning and lather performance, and generally ranges from about 2 wt% to about 50 wt%, from about 5 wt% to about 30 wt%, from about 8 wt% to about 25 wt%, from about 10 wt% to about 20 wt%, about 5 wt%, about 10 wt%, about 12 wt%, about 15 wt%, about 17 wt%, about 18 wt%, or about 20 wt%.

Anionic surfactants suitable for use in the compositions are the alkyl and alkyl ether sulfates. Other suitable anionic surfactants are the water-soluble salts of organic, sulfuric acid reaction products. Still other suitable anionic surfactants are the reaction products of fatty acids esterified with isethionic acid and neutralized with sodium hydroxide. Other similar anionic surfactants are described in U.S. Patent Nos. 2,486,921; 2,486,922; and 2,396,278.

Exemplary anionic surfactants for use in the shampoo composition include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium cocoyl isethionate and combinations thereof. In a further embodiment, the anionic surfactant is sodium lauryl sulfate or sodium laureth sulfate.

Suitable amphoteric or zwitterionic surfactants for use in the shampoo composition herein include those which are known for use in shampoo or other personal care cleansing. Concentrations of such amphoteric surfactants range from about 0.5 wt% to about 20 wt%, and from about 1 wt% to about 10 wt%. Non limiting examples of suitable zwitterionic or amphoteric surfactants are described in U.S. Patent Nos. 5,104,646 and 5,106,609.

Amphoteric detersive surfactants suitable for use in the shampoo composition include those surfactants broadly described as derivatives of aliphatic secondary and tertiary amines in which the aliphatic radical can be straight or branched chain and wherein one of the aliphatic substituents contains from about 8 to about 18 carbon atoms and one contains an anionic group such as carboxy, sulfonate, sulfate, phosphate, or phosphonate. Exemplary amphoteric detersive surfactants for use in the present shampoo composition include cocoamphoacetate, cocoamphodiacetate, lauroamphoacetate, lauroamphodiacetate, and mixtures thereof.

Zwitterionic detersive surfactants suitable for use in the shampoo composition include those surfactants broadly described as derivatives of aliphatic quaternaryammonium, phosphonium, and sulfonium compounds, in which the aliphatic radicals can be straight or branched chain, and wherein one of the aliphatic substituents contains from about 8 to about 18 carbon atoms and one contains an anionic group such as carboxy, sulfonate, sulfate, phosphate or phosphonate. In another embodiment, zwitterionics such as betaines are selected.

Non limiting examples of other anionic, zwitterionic, amphoteric or optional additional surfactants suitable for use in the shampoo composition are described in McCutcheon's, Emulsifiers and Detergents, 1989 Annual, published by M. C. Publishing Co., and U.S. Patent Nos. 3,929,678, 2,658,072; 2,438,091; 2,528,378.

The shampoo composition may also comprise a shampoo gel matrix, an aqueous carrier, and other additional ingredients described herein.

### B. AQUEOUS CARRIER

The shampoo composition may comprise a first aqueous carrier. Accordingly, the formulations of the shampoo composition can be in the form of pourable liquids (under ambient conditions). Such compositions will therefore typically comprise a first aqueous carrier, which is present at a level of at least 20 wt%, from about 20 wt% to about 95 wt%, or from about 60 wt% to about 85 wt%. The first aqueous carrier may comprise water, or a miscible mixture of water and organic solvent, and in one aspect may comprise water with minimal or no significant concentrations of organic solvent, except as otherwise incidentally incorporated into the composition as minor ingredients of other components.

The first aqueous carriers useful in the shampoo composition include water and water solutions of lower alkyl alcohols and polyhydric alcohols. The lower alkyl alcohols useful herein are monohydric alcohols having 1 to 6 carbons, in one aspect, ethanol and isopropanol. The polyhydric alcohols useful herein include propylene glycol, hexylene glycol, glycerin, and propane diol.

### C. SHAMPOO GEL MATRIX

The shampoo composition described herein may comprise a shampoo gel matrix. The shampoo gel matrix comprises (i) from about 0.1% to about 20% of one or more fatty alcohols, alternative from about 0.5% to about 14%, alternatively from about 1% to about 10%, alternatively from about 6% to about 8%, by weight of the shampoo gel matrix; (ii) from about 0.1% to about 10% of one or more shampoo gel matrix surfactants, by weight of the shampoo gel matrix; and (iii) from about 20% to about 95% of an aqueous carrier, alternatively from about 60% to about 85% by weight of the shampoo gel matrix.

The fatty alcohols useful herein are those having from about 10 to about 40 carbon atoms, from about 12 to about 22 carbon atoms, from about 16 to about 22 carbon atoms, or about 16 to about 18 carbon atoms. These fatty alcohols can be straight or branched chain alcohols and can be saturated or unsaturated. Nonlimiting examples of fatty alcohols include, cetyl alcohol, stearyl alcohol, behenyl alcohol, and mixtures thereof. Mixtures of cetyl and stearyl alcohol in a ratio of from about 20:80 to about 80:20 are suitable.

The shampoo gel matrix surfactants may be any of the detersive surfactants described in section "A" herein.

The aqueous carrier may comprise water, or a miscible mixture of water and organic solvent, and in one aspect may comprise water with minimal or no significant concentrations of organic solvent, except as otherwise incidentally incorporated into the composition as minor ingredients of other components.

The aqueous carrier useful herein includes water and water solutions of lower alkyl alcohols and polyhydric alcohols. The lower alkyl alcohols useful herein are monohydric alcohols having 1 to 6 carbons, in one aspect, ethanol and isopropanol. Exemplary polyhydric alcohols useful herein include propylene glycol, hexylene glycol, glycerin, and propane diol.

### D. ADDITIONAL COMPONENTS

The shampoo compositions of the present invention may optionally comprise one or more additional components known for use in hair care or personal care products, provided that the additional components are physically and chemically compatible with the essential components described herein, or do not otherwise unduly impair product stability, aesthetics or performance. Such additional components are most typically those described in reference books such as the CTFA Cosmetic Ingredient Handbook, Second Edition, The Cosmetic, Toiletries, and Fragrance Association, Inc. 1988, 1992. Individual concentrations of such additional components may range from about 0.001 wt% to about 10 wt% by weight of the hair care compositions.

Non-limiting examples of additional components for use in the hair care compositions include conditioning agents , natural cationic deposition polymers, synthetic cationic deposition polymers, anti-dandruff agents, particles, suspending agents, paraffinic hydrocarbons, propellants, viscosity modifiers, dyes, non-volatile solvents or diluents (water-soluble and water-insoluble), pearlescent aids, foam boosters, additional surfactants or nonionic cosurfactants, pediculocides, pH adjusting agents, perfumes, preservatives, proteins, skin active agents, sunscreens, UV absorbers, and vitamins.

### 1. Conditioning Agent

The hair care compositions may comprise one or more conditioning agents. Conditioning agents include materials that are used to give a particular conditioning benefit to hair. The conditioning agents useful in the hair care compositions of the present invention typically comprise a water-insoluble, water-dispersible, non-volatile, liquid that forms emulsified, liquid particles. Suitable conditioning agents for use in the hair care composition are those conditioning agents characterized generally as silicones , organic conditioning oils or combinations thereof, or those conditioning agents which otherwise form liquid, dispersed particles in the aqueous surfactant matrix.

One or more conditioning agents are present from about 0.01 wt% to about 10 wt%, from about 0.1 wt% to about 8 wt%, and from about 0.2 wt% to about 4 wt%, by weight of the composition.

### Silicone Conditioning Agent

The compositions of the present invention may contain one or more silicone conditioning agents. Examples of the silicones include dimethicones, dimethiconols, cyclic silicones, methylphenyl polysiloxane, and modified silicones with various functional groups such as amino groups, quaternary ammonium salt groups, aliphatic groups, alcohol groups, carboxylic acid groups, ether groups, epoxy groups, sugar or polysaccharide groups, fluorine-modified alkyl groups, alkoxy groups, or combinations of such groups. Such silicones may be soluble or insoluble in the aqueous (or non-aqueous) product carrier. In the case of insoluble liquid silicones, the polymer can be in an emulsified form with droplet size of about 10 nm to about 30 micrometers

Suitable silicone conditioning agents include durable silicone materials such as cross-linkable silicone compounds containing different functional groups including siloxanes or silsequioxanes with terminal hydroxyl or alkoxy function groups. Non-limiting examples include Wacker Belsil ADM 8301E and Belsil ADM 6300E. Other suitable durable conditioning compounds include cross-linkable silicones such as MQ-resin, amino fluids and mixture thereof. Non-limiting examples include Wacker ADM 8500E, Dow Corning DX AP6087, Momentive Silform flexible resins, SR1000 MQ-resin and mxture thereof. Such compounds can cross-link upon drying on hair surface or after exposing to heat treatment to impart durable conditioning over multiple washing cycles.

### Organic Conditioning Materials

The conditioning agent of the compositions of the present invention may also comprise at least one organic conditioning material such as oil or wax, either alone or in combination with other conditioning agents, such as the silicones described above. The organic material can be nonpolymeric, oligomeric or polymeric. It may be in the form of oil or wax and may be added in the formulation neat or in a pre-emulsified form. Some non-limiting examples of organic conditioning materials include, but are not limited to: i) hydrocarbon oils; ii) polyolefins, iii) fatty esters, iv) fluorinated conditioning compounds, v) fatty alcohols, vi) alkyl glucosides and alkyl glucoside derivatives; vii) quaternary ammonium compounds; viii) polyethylene glycols and polypropylene glycols having a molecular weight of up to about 2,000,000 including those with CTFA names PEG-20 200, PEG-400, PEG-600, PEG-1000, PEG-2M, PEG-7M, PEG-14M, PEG-45M and mixtures thereof.

### 2. Deposition Polymer

The shampoo composition of the present invention may also comprise a cationic deposition polymer. These cationic deposition polymers can include at least one of (a) a cationic guar polymer, (b) a cationic non-guar galactomannan polymer, (c) a cationic tapioca polymer, (d) a cationic copolymer of acrylamide monomers and cationic monomers, and/or (e) a synthetic, non-crosslinked, cationic polymer, which may or may not form lyotropic liquid crystals upon combination with the detersive surfactant (f) a cationic cellulose polymer. Additionally, the cationic deposition polymer can be a mixture of deposition polymers.

### 3. Rheology Modifier

In one embodiment the shampoo composition may include one or more rheology modifiers to adjust the rheological characteristics of the composition for better feel, in-use properties and the suspending stability of the composition. For example, the rheological properties are adjusted so that the composition remains uniform during its storage and transportation and it does not drip undesirably onto other areas of the body, clothing or home furnishings during its use. Any suitable rheology modifier can be used. In an embodiment, the leave-on treatment may comprise from about 0.01% to about 3% of a rheology modifier, alternatively from about 0.1% to about 1% of a rheology modifier,

The one or more rheology modifier may be selected from the group consisting of polyacrylamide thickeners, cationically modified polysaccharides, associative thickeners, and mixtures thereof. Associative thickeners include a variety of material classes such as, for example: hydrophobically modified cellulose derivatives; hydrophobically modified alkoxylated urethane polymers, such as PEG-150/decyl alcohol/SMDI copolymer, PEG-150/stearyl alcohol/SMDI copolymer, polyurethane-39; hydrophobically modified, alkali swellable emulsions, such as hydrophobically modified polypolyacrylates, hydrophobically modified polyacrylic acids, and hydrophobically modified polyacrylamides; hydrophobically modified polyethers. These materials may have a hydrophobe that can be selected from cetyl, stearyl, oleayl, and combinations thereof, and a hydrophilic portion of repeating ethylene oxide groups with repeat units from 10-300, alternatively from 30-200, and alternatively from 40-150. Examples of this class include PEG-120-methylglucose dioleate, PEG-(40 or 60) sorbitan tetraoleate, PEG-150 pentaerythrityl tetrastearate, PEG-55 propylene glycol oleate, PEG-150 distearate.

Non-limiting examples of additional rheology modifiers include acrylamide/ammonium acrylate copolymer (and)polyisobutene (and) polysorbate 20; acrylamide/sodium acryloyldimethyl taurate copolymer/ isohexadecane/ polysorbate 80; acrylates copolymer; acrylates/beheneth-25 methacrylate copolymer; acrylates/C10-C30 alkyl acrylate crosspolymer; acrylates/steareth-20 itaconate copolymer; ammonium polyacrylate/Isohexadecane/PEG-40 castor oil; C12-16 alkyl PEG-2 hydroxypropylhydroxyethyl ethylcellulose (HM-EHEC); carbomer; crosslinked polyvinylpyrrolidone (PVP); dibenzylidene sorbitol; hydroxyethyl ethylcellulose (EHEC); hydroxypropyl methylcellulose (HPMC); hydroxypropyl methylcellulose (HPMC); hydroxypropylcellulose (HPC); methylcellulose (MC); methylhydroxyethyl cellulose (MEHEC); PEG-150/decyl alcohol/SMDI copolymer; PEG-150/stearyl alcohol/SMDI copolymer; polyacrylamide/C13-14 isoparaffin/laureth-7; polyacrylate 13/polyisobutene/polysorbate 20; polyacrylate crosspolymer-6; polyamide-3; polyquaternium-37 (and) hydrogenated polydecene (and) trideceth-6; polyurethane-39; sodium acrylate/acryloyldimethyltaurate/dimethylacrylamide; crosspolymer (and) isohexadecane (and) polysorbate 60; sodium polyacrylate. Exemplary commercially-available rheology modifiers include ACULYN™ 28, Klucel M CS, Klucel H CS, Klucel G CS, SYLVACLEAR AF1900V, SYLVACLEAR PA1200V, Benecel E10M, Benecel K35M, Optasense RMC70, ACULYN™33, ACULYN™46, ACULYN™22, ACULYN™44, Carbopol Ultrez 20, Carbopol Ultrez 21, Carbopol Ultrez 10, Carbopol 1342, Sepigel™ 305, Simulgel™600, Sepimax Zen, and/or combinations thereof.

### 4. Benefit Agents

In an embodiment, the shampoo composition further comprises one or more additional benefit agents. The benefit agents comprise a material selected from the group consisting of anti-fungal agents, anti-itch agents, anti-bacterial agents, anti-microbial agents, moisturization agents, anti-oxidants, chelants, vitamins, lipid soluble vitamins, perfumes, brighteners, enzymes, sensates, attractants, dyes, pigments, bleaches, and mixtures thereof.

According to an embodiment, the shampoo composition comprises an anti-dandruff active, which may be an anti-dandruff active particulate. The anti-dandruff active can be selected from the group consisting of: pyridinethione salts; azoles, such as an imidazole such as ketoconazole, econazole, climbazole and elubiol; selenium sulphide; coal tar, particulate sulfur; keratolytic agents such as salicylic acid; and mixtures thereof. In an embodiment, the anti-dandruff particulate is a pyridinethione salt.

Pyridinethione particulates are suitable particulate anti-dandruff actives. In an embodiment, the anti-dandruff active is a 1-hydroxy-2-pyridinethione salt and is in particulate form. In an embodiment, the concentration of pyridinethione anti-dandruff particulate ranges from about 0.01 wt% to about 5 wt%, or from about 0.1 wt% to about 3 wt%, or from about 0.1 wt% to about 2 wt%. In an embodiment, the pyridinethione salts are those formed from heavy metals such as zinc, tin, cadmium, magnesium, aluminium and zirconium, generally zinc, typically the zinc salt of 1-hydroxy-2-pyridinethione (known as "zinc pyridinethione" or "ZPT"), commonly 1-hydroxy-2-pyridinethione salts in platelet particle form. In an embodiment, the 1-hydroxy-2-pyridinethione salts in platelet particle form have an average particle size of up to about 20 microns, or up to about 5 microns, or up to about 2.5 microns. Salts formed from other cations, such as sodium, may also be suitable. Pyridinethione anti-dandruff actives are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982.

The anti-dandruff active can also be selected from polyvalent metal salts of pyrithione, the composition further comprises one or more anti-fungal and/or anti-microbial actives. Embodiments of the present invention may also comprise a combination of anti-microbial actives.

In an embodiment, the composition comprises an effective amount of a zinc-containing layered materials (ZLMs). Another common class of ZLMs, which are often, but not always, synthetic, is layered double hydroxides. In an embodiment, the ZLM is a layered double hydroxide conforming to the formula [M²⁺₁₋ₓM³⁺ₓ(OH)₂]^{x+} A^{m-}_{x/m}·nH₂O wherein some or all of the divalent ions (M²⁺) are zinc ions (Crepaldi, EL, Pava, PC, Tronto, J, Valim, JB J. Colloid Interfac. Sci. 2002, 248, 429-42).

Yet another class of ZLMs can be prepared called hydroxy double salts (Morioka, H., Tagaya, H., Karasu, M, Kadokawa, J, Chiba, K Inorg. Chem. 1999, 38, 4211-6). In an embodiment, the ZLM is a hydroxy double salt conforming to the formula [M²⁺₁₋ₓM²⁺₁₊ₓ(OH)_{3(1-y})]⁺ Aⁿ⁻_{(1=3y)/n}·nH₂O where the two metal ions (M²⁺) may be the same or different. If they are the same and represented by zinc, the formula simplifies to [Zn₁₊ₓ(OH)₂]^{2x+} 2x A⁻·nH₂O. This latter formula represents (where x=0.4) materials such as zinc hydroxychloride and zinc hydroxynitrate. In an embodiment, the ZLM is zinc hydroxychloride and/or zinc hydroxynitrate. These are related to hydrozincite as well wherein a divalent anion replace the monovalent anion. These materials can also be formed *in situ* in a composition or in or during a production process.

In an embodiment, the composition comprises basic zinc carbonate. Commercially available sources of basic zinc carbonate include Zinc Carbonate Basic (Cater Chemicals: Bensenville, IL, USA), Zinc Carbonate (Shepherd Chemicals: Norwood, OH, USA), Zinc Carbonate (CPS Union Corp.: New York, NY, USA), Zinc Carbonate (Elementis Pigments: Durham, UK), and Zinc Carbonate AC (Bruggemann Chemical: Newtown Square, PA, USA). Basic zinc carbonate, which also may be referred to commercially as "Zinc Carbonate" or "Zinc Carbonate Basic" or "Zinc Hydroxy Carbonate", is a synthetic version consisting of materials similar to naturally occurring hydrozincite. The idealized stoichiometry is represented by Zn₅(OH)₆(CO₃)₂ but the actual stoichiometric ratios can vary slightly and other impurities may be incorporated in the crystal lattice.

In embodiments having a zinc-containing layered material and a pyrithione or polyvalent metal salt of pyrithione, the ratio of zinc-containing layered material to pyrithione or a polyvalent metal salt of pyrithione is from about 5:100 to about 10:1, or from about 2:10 to about 5:1, or from about 1:2 to about 3:1.

In an embodiment, the composition comprises from about 0.001 wt% to about 10 wt%, or from about 0.01 wt% to about 7 wt%, or from about 0.1 wt% to about 5 wt% of a zinc-containing layered material (ZLMs), by total weight of the composition.

The shampoo compositions of the present invention may be presented in typical hair care formulations. They may be in the form of solutions, dispersion, emulsions, powders, talcs, encapsulated, spheres, spongers, solid dosage forms, foams, and other delivery mechanisms.

According to one embodiment, the hair care compositions may be provided in the form of a porous, dissolvable solid structure, such as those disclosed in U.S. Patent Application Publication Nos. 2009/0232873; and 2010/0179083. Accordingly, the hair care compositions comprise a chelant, a buffer system comprising an organic acid, from about 23% to about 75% surfactant; from about 10% to about 50% water soluble polymer; and optionally, from about 1% to about 15% plasticizer; such that the hair care composition is in the form of a flexible porous dissolvable solid structure, wherein said structure has a Percent open cell content of from about 80% to about 100%.

### EXAMPLES

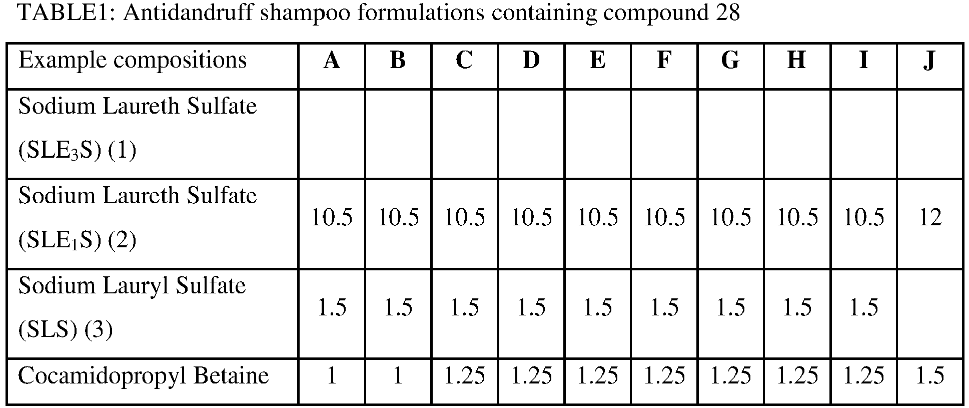

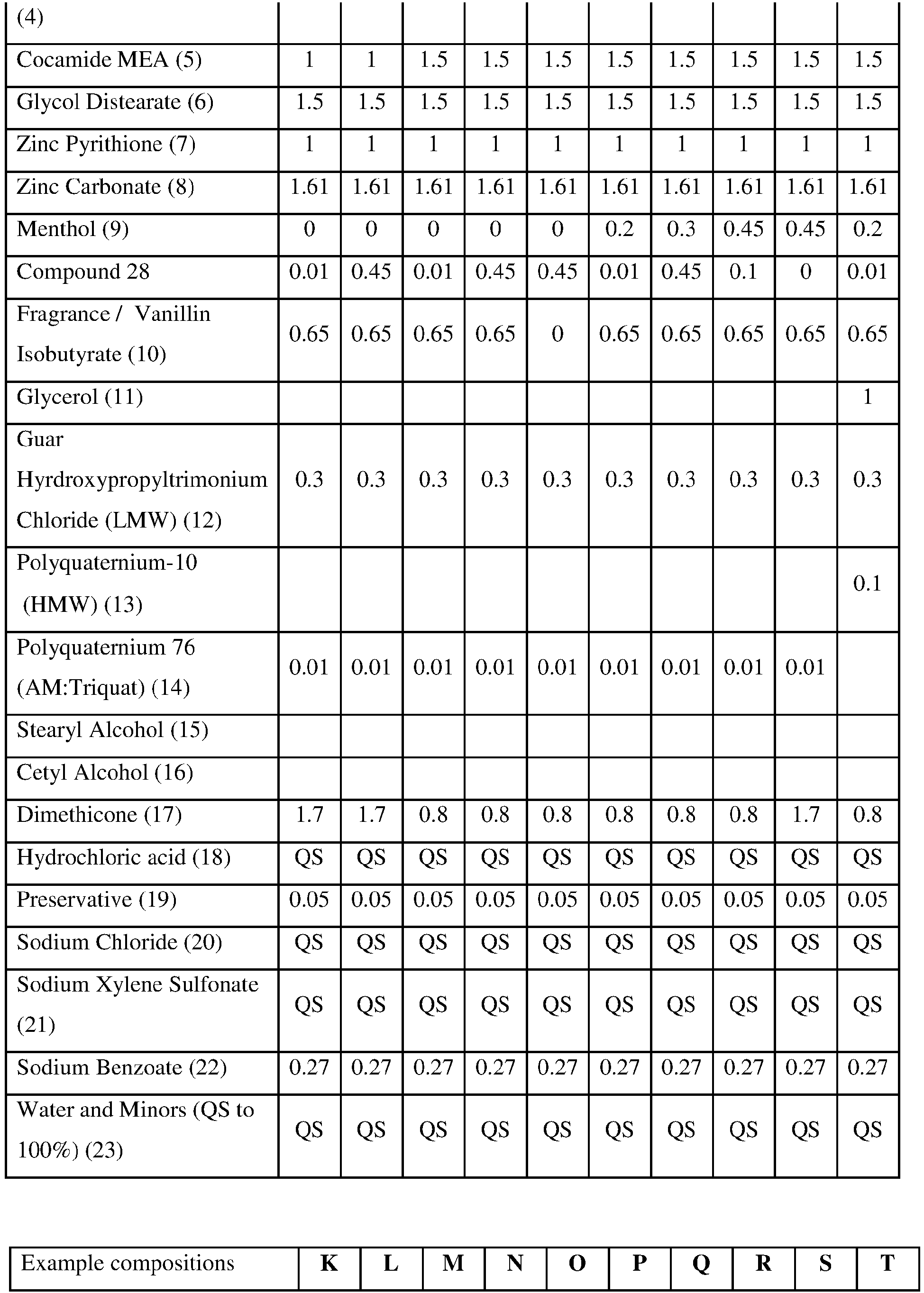

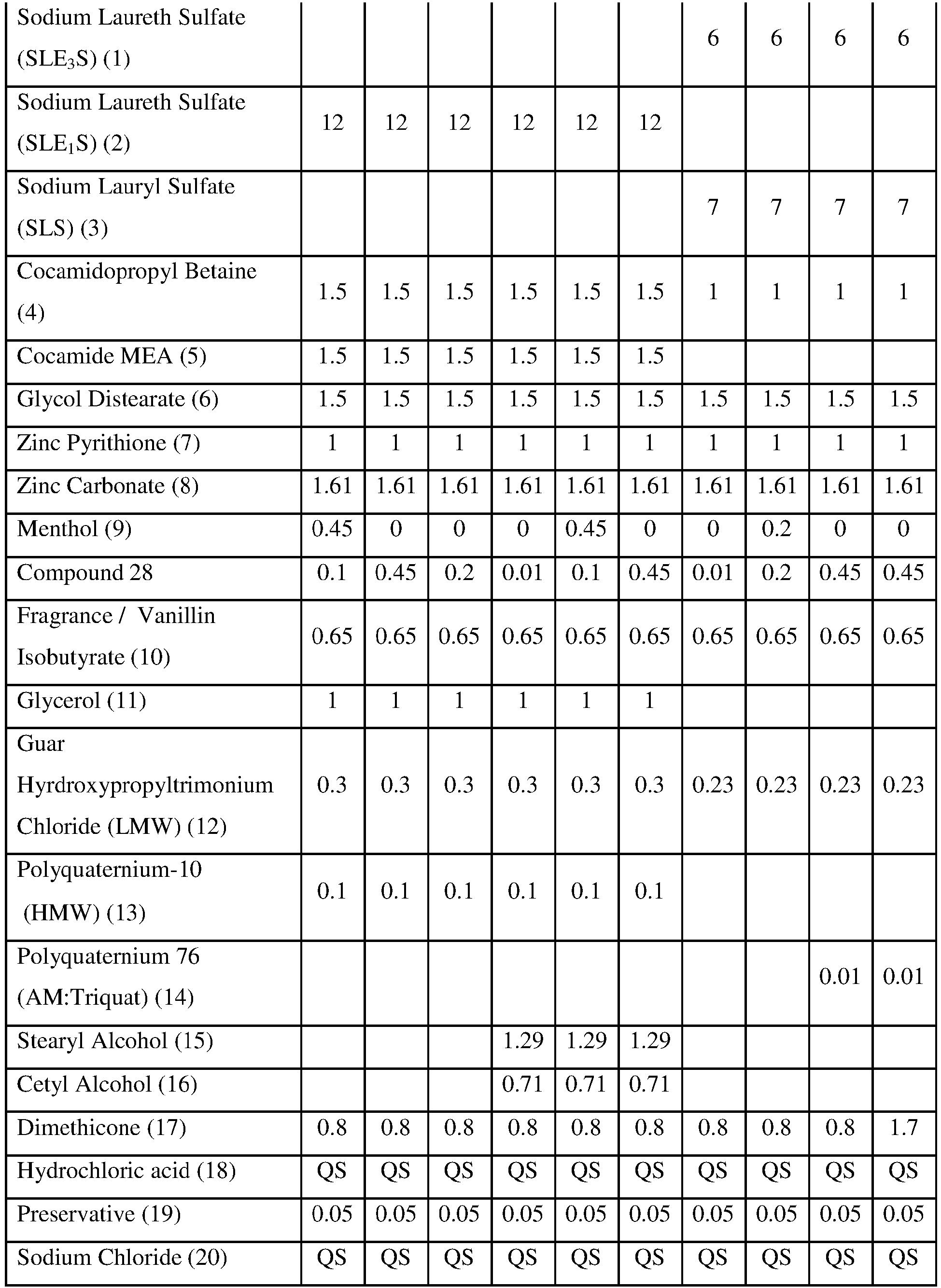

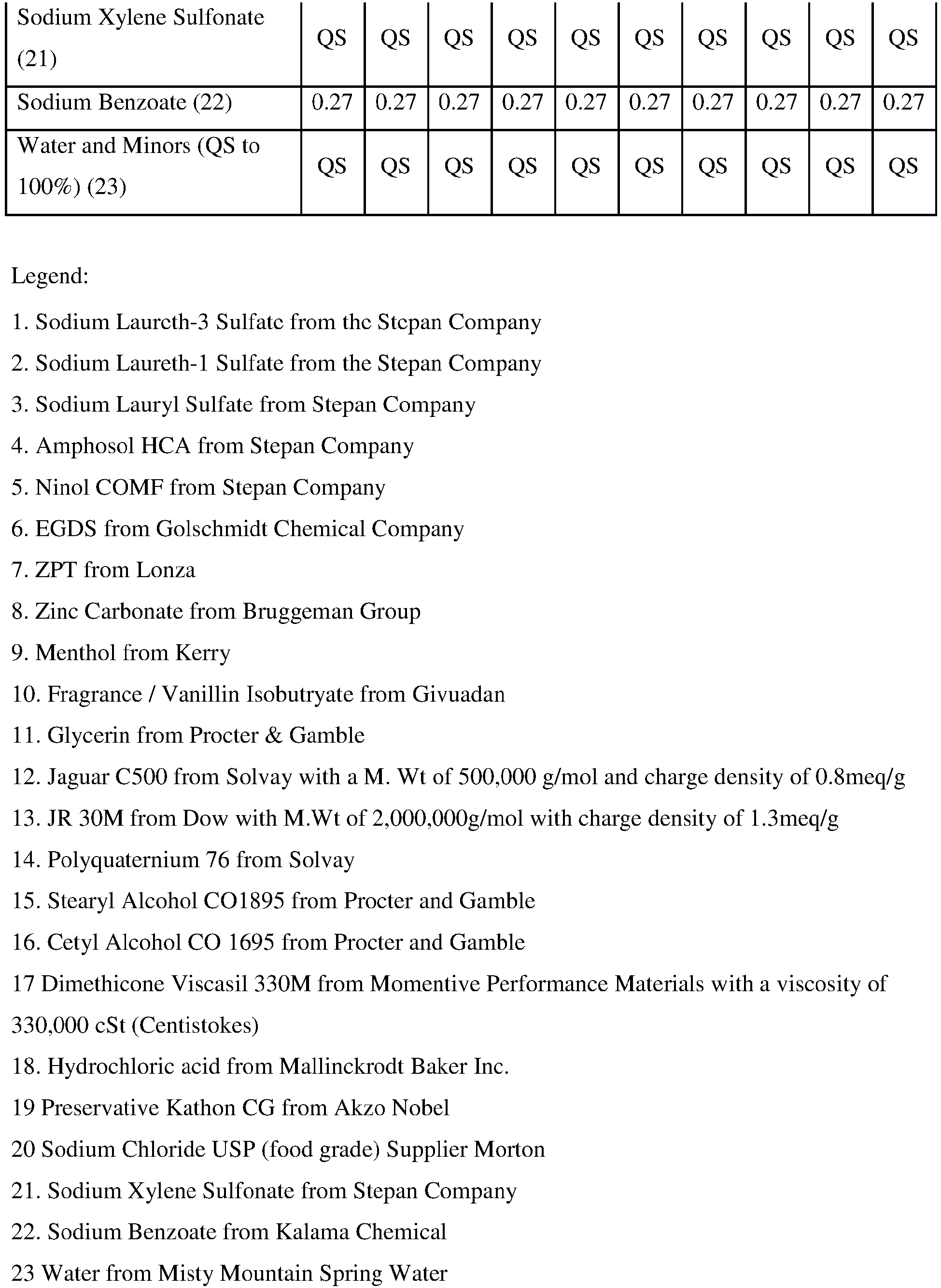

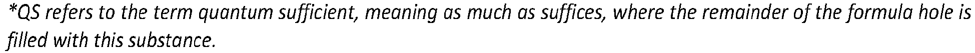

### Making with Compound 28 in shampoo formulations:

Compound 28 is solubilized by adding it to SLS surfactant and Vanillin Isobutyrate oil while heating until visually soluble in solution. Glycerol and heating to 60C can be used to solubilize compound 28 when SLS is not present in the formulation such as in Examples (J-P). Once compound 28 is solubilized it can be added to the shampoo at any time in the making process.

Compound 28 should be preferably made with aldehyde and ketone free perfumes. Compound 28 contains a primary amine off of the alanine. Primary amines can be made into an imine or Schiff base under the right conditions in the presence of an aldehyde or ketone. This possibly could render the Compound 28 less effective. Vanillin Isobutryate is one such material that can be used as a perfume in shampoo formulations as it displays a sweet vanilla, chocolate fragrance.

### Salon panel:

Five panelists for each shampoo leg are investigated (Example D and I). Panelists shampoo via a stylist in a hair care salon with 5ml of shampoo. Example D contains 0.45% Compound 28 and Example I contains 0.45% Menthol. The panelist's heads are washed with shampoo and permitted to style hair if desired. A questionnaire is given to complete throughout the day and the beginning of the next day. Values for each question are scored on a 0-10 scale with 0 being lowest cooling intensity and 10 being most intense cooling.

**TABLE 2: Panelists evaluated cooling intensity**

| Example | Within 10minutes | | | | ∼1-7hr | ∼8-12hr | ∼12-14hr | ∼24hr | |
|---|---|---|---|---|---|---|---|---|---|
| | during Shampoo | during rinse | while towel drying | while styling | throughout the day | end of day | night shower | next morning before shower | in shower next morning |
| D | 3 | 4 | 4 | 5 | 7 | 7 | 6 | 3 | 6 |
| I | 5 | 4 | 4 | 3 | 1 | 0 | 0 | 0 | 0 |

Results shown in TABLE 2 show that panelists that use Example I only report a moderate cooling sensation within the amount of time it takes to shampoo, rinse, dry, and style their hair (10minutes). One panelist reports a low cooling sensation approximately 1 hour after treatment with Example I. Example D is observed to give moderate cooling throughout the shampoo process. Example D is observed to increase in cooling intensity throughout the day and into night (Cooling time 1-14 hours). It is hypothesized that Compound 28 slowly partitions into the skin and reaches maximal effect at approximately 8-12 hours post treatment. It is observed that panelists receiving treatment with Example D also notice low to moderate cooling sensations the following morning before shower and in shower when utilizing their own shampoo (20-24hrs post treatment of Example D). The hair composition of the present invention may provide cooling sensation in the range of from about 1 hour to about 24 hours, in an embodiment, in the range of from about 8 to about 12 hours.

Example D contains 4500ppm of Compound D and Example I contains 4500ppm of Menthol. It is assumed that materials when solubilized have approximate scalp deposition efficiency of 1%. It is believed that based on 1% depo efficiency that the panelists respond to approximately 45ppm or lower on scalp for each example.

## Claims

1. A hair care shampoo composition comprising a compound comprising the following structure: 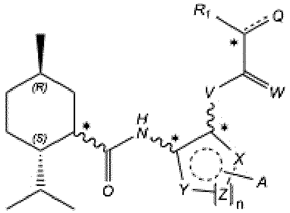
R₁ is selected from H, alkyl, amino alkyl, alkoxy;
Q = H₂, O, -OR₁, -N(R₁)₂, -OPO(OR₁)ₓ, -PO(OR₁)ₓ, -P(OR₁)ₓ where x = 1-2;
V = NR₁, O, -OPO(OR₁)ₓ, -PO(OR₁)ₓ, -P(OR₁)ₓ where x = 1-2;
W = H₂, O;
X, Y = independently selected from H, aryl, naphthyl for n=0;
X, Y = aliphatic CH₂ or aromatic CH for n ≥ 1 and Z is selected from aliphatic CH₂, aromatic CH, or heteroatom;
A = aryl, substituted aryl or fused aryl; and
stereochemistry is variable at the positions marked*.

2. The hair care shampoo composition according to claim 1 wherein the compound is present from 4500 ppm to 100 ppm.

3. The hair care shampoo composition according to any preceding claims wherein the composition provides a cooling sensation in the range of from 1 hour to 24 hours.

4. The hair care shampoo composition according to any preceding claims wherein the composition provides a cooling sensation in the range of from 8 hours to 12 hours.

5. The hair care shampoo composition according to any preceding claims wherein the compound further comprises an antidandruff active.

6. The hair care shampoo composition according to any preceding claims wherein the anti-dandruff active is selected from the group consisting of pyridinethione salts; azoles, selenium sulphide; coal tar, particulate sulfur, keratolytic agents and mixtures thereof.

7. The hair care shampoo composition according to any preceding claims wherein the anti-dandruff active is zinc pyrithione.

8. The hair care shampoo composition according to any preceding claims wherein the composition comprises a detersive surfactant.

9. The hair care shampoo composition according to any preceding claims wherein the composition comprises a benefit agent selected from the group consisting of an anti-fungal agent, anti-itch agent, anti-bacterial agent, anti-microbial agent, moisturization agent, anti-oxidant, vitamin, lipid soluble vitamin, chelant, perfume, brightener, enzyme, sensate, attractant, dyes, pigment, bleaches, and mixtures thereof.

10. The hair care shampoo composition according to any preceding claims wherein the composition comprises a deposition polymer.

11. The hair care shampoo composition according to any preceding claims further comprising a perfume which is free of aldehydes and ketones.

12. The hair care shampoo composition according to any preceding claims wherein the composition comprises a conditioning agent.

13. The hair care shampoo composition according to any preceding claims wherein the composition comprises zinc carbonate.

14. A hair care shampoo composition according to any preceding claims comprising a compound comprising the following structure: 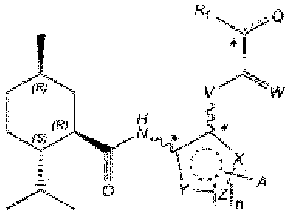
R₁ is selected from H, alkyl, amino alkyl, alkoxy;
Q = H₂, O, -OR₁, -N(R₁)₂, -OPO(OR₁)ₓ, -PO(OR₁)ₓ, -P(OR₁)ₓ where x = 1-2;
V = NR₁, O, -OPO(OR₁)ₓ, -PO(OR₁)ₓ, -P(OR₁)ₓ where x = 1-2;
W = H₂, O;
X, Y = independently selected from H, aryl, naphthyl for n=0;
X, Y = aliphatic CH₂ or aromatic CH for n ≥ 1 and Z is selected from aliphatic CH₂, aromatic CH, or heteroatom;
A = aryl, substituted aryl or fused aryl; and
stereochemistry is variable at the positions marked*.

15. A hair care shampoo composition according to any preceding claims comprising a compound comprising the following structure: 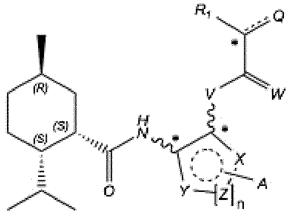
R₁ is selected from H, alkyl, amino alkyl, alkoxy;
Q = H₂, O, -OR₁, -N(R₁)₂, -OPO(OR₁)ₓ, -PO(OR₁)ₓ, -P(OR₁)ₓ where x = 1-2;
V = NR₁, O, -OPO(OR₁)ₓ, -PO(OR₁)ₓ, -P(OR₁)ₓ where x = 1-2;
W = H₂, O;
X, Y = independently selected from H, aryl, naphthyl for n=0;
X, Y = aliphatic CH₂ or aromatic CH for n ≥ 1 and Z is selected from aliphatic CH₂, aromatic CH, or heteroatom;
A = aryl, substituted aryl or fused aryl; and
stereochemistry is variable at the positions marked*.

## Patentansprüche

1. Haarpflegeshampoozusammensetzung, umfassend eine Verbindung, umfassend die folgende Struktur: 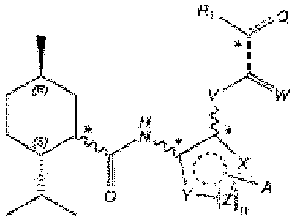
worin R₁ ausgewählt ist aus H, Alkyl, Aminoalkyl, Alkoxy;
Q = H₂, O, -OR₁, -N(R₁)₂, -OPO(OR₁)ₓ, -PO(OR₁)ₓ, -P(OR₁)ₓ, worin x = 1-2;
V = NR₁, O, -OPO(OR₁)ₓ, -PO(OR₁)ₓ, -P(OR₁)ₓ, worin x = 1-2;
W = H₂, O;
X, Y = unabhängig ausgewählt aus H, Aryl, Naphthyl für n = 0;
X, Y = aliphatisches CH₂ oder aromatisches CH für n ≥ 1 und Z ausgewählt ist aus aliphatischem CH₂, aromatischem CH oder Heteroatom;
A = Aryl, substituiertes Aryl oder kondensiertes Aryl; und
die Stereochemie an den mit * markierten Positionen variabel ist.

2. Haarpflegeshampoozusammensetzung nach Anspruch 1, wobei die Verbindung von 4500 ppm bis 100 ppm vorhanden ist.

3. Haarpflegeshampoozusammensetzung nach einem der vorstehenden Ansprüche, wobei die Zusammensetzung im Bereich von 1 Stunde bis 24 Stunden ein Abkühlempfinden bereitstellt.

4. Haarpflegeshampoozusammensetzung nach einem der vorstehenden Ansprüche, wobei die Zusammensetzung im Bereich von 8 Stunden bis 12 Stunden ein Abkühlempfinden bereitstellt.

5. Haarpflegeshampoozusammensetzung nach einem der vorstehenden Ansprüche, wobei die Verbindung ferner einen Antischuppenwirkstoff umfasst.

6. Haarpflegeshampoozusammensetzung nach einem der vorstehenden Ansprüche, wobei der Antischuppenwirkstoff ausgewählt ist aus der Gruppe, bestehend aus Pyridinthionsalzen; Azolen, Selensulfid; Kohlenteer, teilchenförmigem Schwefel, keratolytischen Mitteln und Mischungen davon.

7. Haarpflegeshampoozusammensetzung nach einem der vorstehenden Ansprüche, wobei der Antischuppenwirkstoff Zinkpyrithion ist.

8. Haarpflegeshampoozusammensetzung nach einem der vorstehenden Ansprüche, wobei die Zusammensetzung ein Reinigungstensid umfasst.

9. Haarpflegeshampoozusammensetzung nach einem der vorstehenden Ansprüche, wobei die Zusammensetzung einen Wirkstoff umfasst, ausgewählt aus der Gruppe, bestehend aus einem antimykotischen Mittel, juckreizhemmenden Mittel, antibakteriellen Mittel, antimikrobiellen Mittel, Befeuchtungsmittel, Antioxidationsmittel, Vitamin, lipidlöslichen Vitamin, Chelant, Duftstoff, Aufheller, Enzym, einer sinnlich wahrgenommenen Substanz, einem Lockstoff, Farbstoffen, einem Pigment, Bleichmittel und Mischungen davon.

10. Haarpflegeshampoozusammensetzung nach einem der vorstehenden Ansprüche, wobei die Zusammensetzung ein Abscheidepolymer umfasst.

11. Haarpflegeshampoozusammensetzung nach einem der vorstehenden Ansprüche, ferner umfassend einen Duftstoff, der frei von Aldehyden und Ketonen ist.

12. Haarpflegeshampoozusammensetzung nach einem der vorstehenden Ansprüche, wobei die Zusammensetzung ein Konditioniermittel umfasst.

13. Haarpflegeshampoozusammensetzung nach einem der vorstehenden Ansprüche, wobei die Zusammensetzung Zinkcarbonat umfasst.

14. Haarpflegeshampoozusammensetzung nach einem der vorstehenden Ansprüche, umfassend eine Verbindung, umfassend die folgende Struktur: 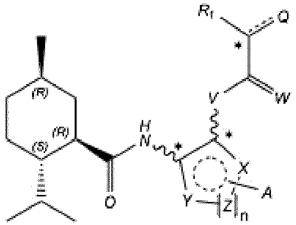
worin R₁ ausgewählt ist aus H, Alkyl, Aminoalkyl, Alkoxy;
Q = H₂, O, -OR₁, -N(R₁)₂, -OPO(OR₁)ₓ, -PO(OR₁)ₓ, -P(OR₁)ₓ, worin x = 1-2;
V = NR₁, O, -OPO(OR₁)ₓ, -PO(OR₁)ₓ, -P(OR₁)ₓ, worin x = 1-2;
W = H₂, O;
X, Y = unabhängig ausgewählt aus H, Aryl, Naphthyl für n = 0;
X, Y = aliphatisches CH₂ oder aromatisches CH für n ≥ 1 und Z ausgewählt ist aus aliphatischem CH₂, aromatischem CH oder Heteroatom;
A = Aryl, substituiertes Aryl oder kondensiertes Aryl; und
die Stereochemie an den mit * markierten Positionen variabel ist.

15. Haarpflegeshampoozusammensetzung nach einem der vorstehenden Ansprüche, umfassend eine Verbindung, umfassend die folgende Struktur: 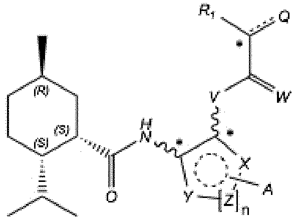
worin R₁ ausgewählt ist aus H, Alkyl, Aminoalkyl, Alkoxy;
Q = H₂, O, -OR₁, -N(R₁)₂, -OPO(OR₁)ₓ, -PO(OR₁)ₓ, -P(OR₁)ₓ, worin x = 1-2;
V = NR₁, O, -OPO(OR₁)ₓ, -PO(OR₁)ₓ, -P(OR₁)ₓ, worin x = 1-2;
W = H₂, O;
X, Y = unabhängig ausgewählt aus H, Aryl, Naphthyl für n = 0;
X, Y = aliphatisches CH₂ oder aromatisches CH für n ≥ 1 und Z ausgewählt ist aus aliphatischem CH₂, aromatischem CH oder Heteroatom;
A = Aryl, substituiertes Aryl oder kondensiertes Aryl; und
die Stereochemie an den mit * markierten Positionen variabel ist.

## Revendications

1. Composition de shampoing de soins capillaires comprenant un composé comprenant la structure suivante : 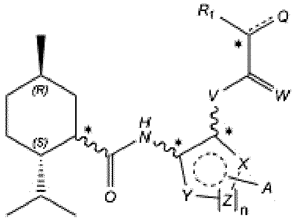
R₁ est choisi parmi H, alkyle, aminoalkyle, alcoxy ;
Q = H₂, O, -OR₁, -N(R₁)₂, -OPO(OR₁)ₓ, -PO(OR₁)ₓ, -P(OR₁)ₓ où x = 1-2 ;
V = NR₁, O, -OPO(OR₁)ₓ, -PO(OR₁)ₓ, -P(OR₁)ₓ où x = 1-2 ;
W = H₂, O ;
X, Y = indépendamment choisi parmi H, aryle, naphtyle pour n=0 ;
X, Y = CH₂ aliphatique ou CH aromatique pour n ≥ 1 et Z est choisi parmi CH₂ aliphatique, CH aromatique, ou hétéroatome ;
A = aryle, aryle substitué ou aryle condensé ; et
la stéréochimie est variable aux positions marquées*.

2. Composition de shampoing de soins capillaires selon la revendication 1 dans laquelle le composé est présent à raison de 4500 ppm à 100 ppm.

3. Composition de shampoing de soins capillaires selon l'une quelconque des revendications précédentes dans laquelle la composition fournit une sensation de rafraîchissement dans la plage allant de 1 heure à 24 heures.

4. Composition de shampoing de soins capillaires selon l'une quelconque des revendications précédentes dans laquelle la composition fournit une sensation de rafraîchissement dans la plage allant de 8 heures à 12 heures.

5. Composition de shampoing de soins capillaires selon l'une quelconque des revendications précédentes dans laquelle le composé comprend en outre un agent actif anti-pelliculaire.

6. Composition de shampoing de soins capillaires selon l'une quelconque des revendications précédentes dans laquelle l'agent actif anti-pelliculaire est choisi dans le groupe constitué de sels de pyridinethione ; azoles, sulfure de sélénium ; goudron de houille, soufre particulaire, agents kératolytiques et mélanges de ceux-ci.

7. Composition de shampoing de soins capillaires selon l'une quelconque des revendications précédentes dans laquelle l'agent actif anti-pelliculaire est la pyrithione de zinc.

8. Composition de shampoing de soins capillaires selon l'une quelconque des revendications précédentes dans laquelle la composition comprend un agent tensioactif détersif.

9. Composition de shampoing de soins capillaires selon l'une quelconque des revendications précédentes dans laquelle la composition comprend un agent bénéfique choisi dans le groupe constitué d'agent antifongique, agent antiprurigineux, agent anti-bactérien, agent antimicrobien, agent d'hydratation, antioxydant, vitamine, vitamine liposoluble, agent chélatant, parfum, azurant, enzyme, agent sensoriel, agent attirant, teintures, pigment, agents de blanchiment, et mélanges de ceux-ci.

10. Composition de shampoing de soins capillaires selon l'une quelconque des revendications précédentes dans laquelle la composition comprend un polymère de dépôt.

11. Composition de shampoing de soins capillaires selon l'une quelconque des revendications précédentes comprenant en outre un parfum qui est exempt d'aldéhydes et de cétones.

12. Composition de shampoing de soins capillaires selon l'une quelconque des revendications précédentes dans laquelle la composition comprend un agent de conditionnement.

13. Composition de shampoing de soins capillaires selon l'une quelconque des revendications précédentes dans laquelle la composition comprend du carbonate de zinc.

14. Composition de shampoing de soins capillaires selon l'une quelconque des revendications précédentes comprenant un composé comprenant la structure suivante : 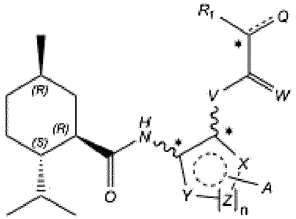
R₁ est choisi parmi H, alkyle, aminoalkyle, alcoxy ;
Q = H₂, O, -OR₁, -N(R₁)₂, -OPO(OR₁)ₓ, -PO(OR₁)ₓ, -P(OR₁)ₓ où x = 1-2 ;
V = NR₁, O, -OPO(OR₁)ₓ, -PO(OR₁)ₓ, -P(OR₁)ₓ où x = 1-2 ;
W = H₂, O ;
X, Y = indépendamment choisi parmi H, aryle, naphtyle pour n=0 ;
X, Y = CH₂ aliphatique ou CH aromatique pour n ≥ 1 et Z est choisi parmi CH₂ aliphatique, CH aromatique, ou hétéroatome ;
A = aryle, aryle substitué ou aryle condensé ; et
la stéréochimie est variable aux positions marquées*.

15. Composition de shampoing de soins capillaires selon l'une quelconque des revendications précédentes comprenant un composé comprenant la structure suivante : 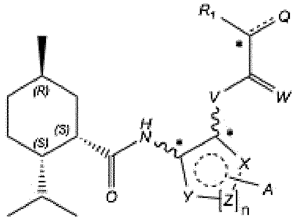
R₁ est choisi parmi H, alkyle, aminoalkyle, alcoxy ;
Q = H₂, O, -OR₁, -N(R₁)₂, -OPO(OR₁)ₓ, -PO(OR₁)ₓ, -P(OR₁)ₓ où x = 1-2 ;
V = NR₁, O, -OPO(OR₁)ₓ, -PO(OR₁)ₓ, -P(OR₁)ₓ où x = 1-2 ;
W = H₂, O ;
X, Y = indépendamment choisi parmi H, aryle, naphtyle pour n=0 ;
X, Y = CH₂ aliphatique ou CH aromatique pour n ≥ 1 et Z est choisi parmi CH₂ aliphatique, CH aromatique, ou hétéroatome ;
A = aryle, aryle substitué ou aryle condensé ; et
la stéréochimie est variable aux positions marquées*.
